# EUROPEAN PATENT APPLICATION

(11) **EP 2 002 812 A1**
(43) Date of publication of application: **17.12.2008**
(21) Application number: 08102345.9
(22) Date of filing: 06.03.2008
(51) Int. Cl.: A61F 9/007, A61M 1/00

(54) **Self sealing cannula/aperture closure cannula**

(30) Priority: 13.06.2007 US 762238
(71) Applicant: Alcon, Inc., 6331 Hünenberg (CH)
(72) Inventor: Hickingbotham, Dyson W., Stouchsberg, PA 19567 (US)
(74) Representative: Hanna, Peter William Derek

(57) **Abstract**

A cannula (10) is provided having a body (12), a sealing disc (14), and a cap (16). The sealing disc (14) is located within the body and is compressed by the cap. An angled cut (40) in the sealing disc allows microsurgical instruments to be inserted through the cannula into the eye. Upon removal, the cut (40) in the sealing disc closes, preventing the loss of intraocular pressure during ophthalmic surgery.

## Description

This invention relates to ophthalmic surgical equipment and more particularly to posterior segment ophthalmic surgical equipment.

### Background of the Invention

Microsurgical instruments typically are used by surgeons for removal of tissue from delicate and restricted spaces in the human body, particularly in surgery on the eye, and more particularly in procedures for removal of the vitreous body, blood, scar tissue, or the crystalline lens. Such instruments include a control console and a surgical handpiece with which the surgeon dissects and removes the tissue. With respect to posterior segment surgery, the handpiece may be a vitreous cutter probe, a laser probe, or an ultrasonic fragmenter for cutting or fragmenting the tissue and is connected to the control console by a long air- pressure (pneumatic) line and/or power cable, optical cable, or flexible tubes for supplying an infusion fluid to the surgical site and for withdrawing or aspirating fluid and cut/fragmented tissue from the site. The cutting, infusion, and aspiration functions of the handpiece are controlled by the remote control console that not only provides power for the surgical handpiece(s) (e.g., a reciprocating or rotating cutting blade or an ultrasonically vibrated needle), but also controls the flow of infusion fluid and provides a source of vacuum (relative to atmosphere) for the aspiration of fluid and cut/fragmented tissue. The functions of the console are controlled manually by the surgeon, usually by means of a foot-operated switch or proportional control.

During posterior segment surgery, the surgeon typically uses several handpieces or instruments during the procedure. This procedure requires that these instruments be inserted into, and removed out of the incision. This repeated removal and insertion can cause trauma to the eye at the incision site. To address this concern, hubbed cannulae were developed at least by the mid-1980s. These devices consist of a narrow tube with an attached hub. The tube is inserted into an incision in the eye up to the hub, which acts as a stop, preventing the tube from entering the eye completely. Often the hub is stitched to the eye to prevent inadvertent removal. Surgical instruments can be inserted into the eye through the tube, and the tube protects the incision sidewall from repeated contact by the instruments. In addition, the surgeon can use the instrument, by manipulating the instrument when the instrument is inserted into the eye through the tube, to help position the eye during surgery. Disadvantages of prior art cannulae include the height of the projection on the surface of the eye, as well as the lack of any means to control loss of intraocular pressure during instrument exchange or removal. The eye, being a pressurized globe, will expel aqueous or vitreous out of the open cannula when a surgical device is not present. With prior art cannulae, loss of intraocular pressure was prevented by the insertion of a plug or cap into the tube to seal the cannula and prevent the expression of fluid and tissue. This is a time-consuming process that often requires additional instrumentation as well as the assistance of other OR personnel and increases the risk of post-operative infection.

Accordingly, a need continues to exist for a cannula that self seals upon instrument removal, thus eliminating the need for plugs, caps, and the instrumentation required to install and remove these devices. Such a device would reduce the amount of time required for surgical procedures and reduce dependency on other OR personnel.

### Brief Summary of the Invention

The present invention improves upon prior art by providing a cannula that self seals upon instrument removal. The cannula generally consists of a tube and an attached hub. Disposed within the hub is a sealing disc having a cut or slit that allows access to the incision, and closes upon instrument removal to seal the cannula.

Accordingly, an objective of the present invention is to provide a cannula.

Another objective of the present invention is to provide a cannula having a sealing disc that self seals upon instrument exchange or removal.

A further objective of the present invention is to provide a cannula that eliminates the need for plugs, caps, and other sealing instrumentation.

A further objective of the present invention is to provide a cannula having a low profile projection on the surface of the eye.

Other objectives, features and advantages of the present invention will become apparent with reference to the drawings, and the following description of the drawings and claims.

### Brief Description of the Drawings

FIG. 1 is an exploded top perspective view of a first embodiment of the cannula of the present invention.
FIG. 2 is a top perspective view of a first embodiment of the cannula of the present invention.
FIG. 3 is an enlarged cross sectional view of a first embodiment of the cannula of the present invention.
FIG. 4 is an exploded cross sectional view of a first embodiment of the cannula of the present invention.
FIG. 5 is an enlarged cross sectional view of a first embodiment of the cannula of the present invention similar to FIG. 4, but with a surgical instrument inserted into the cannula.
FIG. 6 is an enlarged cross sectional view of a second embodiment of the cannula of the present invention.

### Detailed Description of the Invention

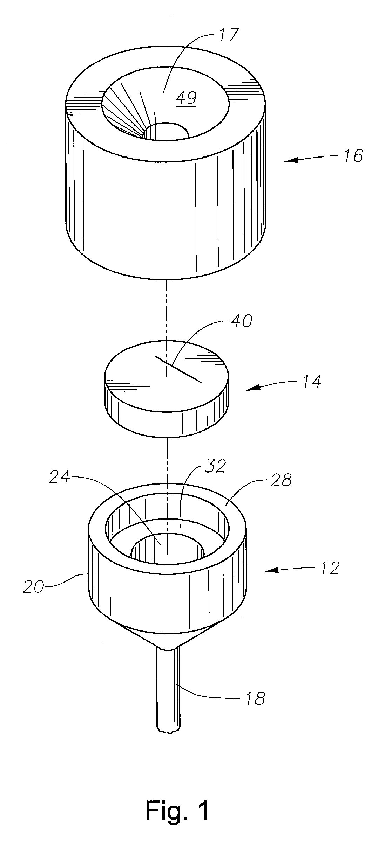

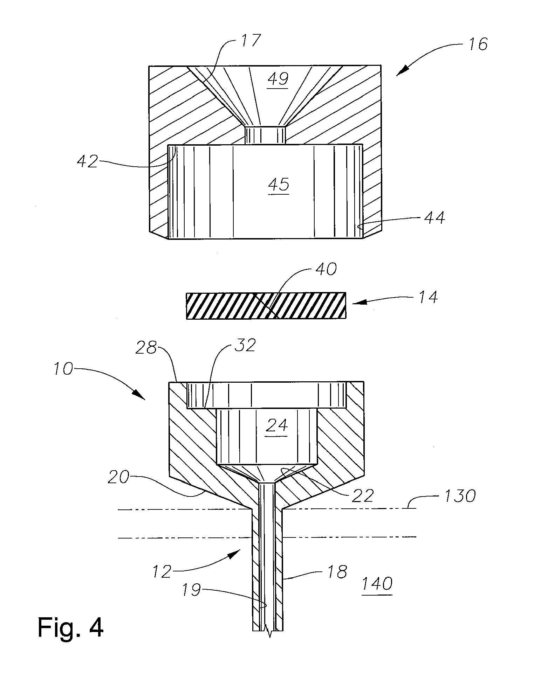

As best seen in FIGS. 1 through 4, cannula 10 generally consists of body 12, sealing disc 14, and cap 16. Body 12 and cap 16 may be made from any suitable material, such as stainless steel, titanium, or thermoplastic. Body 12 is comprised of tube 18 and hub 20 which may be formed integrally or in separate pieces. Tube 18 is of sufficient length to extend through sclera 130 and enter posterior chamber 140. Hub 20 is generally cylindrical with internal cavity 24 having distal floor 22 sloped or tapered at an angle of between about 18-24 degrees (most preferably about 22 degrees) so as to have a funnel shape directed toward bore 19 in tube 18. Cavity 24 may have a diameter of between about 0.040-0.050 inches (most preferably about 0.046 inches) or any other suitable diameter. Cavity 24 generally extends from proximal face 28 to distal floor 22 a depth of between about 0.025 - 0.035 inches (most preferably about 0.029 inches). Proximal face 28 of hub 20 is generally flat with circumferential rabbet 32 recessed into face 28 to a depth of between about 0.005 - 0.015 inches (most preferably about 0.008 inches). Rabbet 32 may have a diameter of between about 0.060 - 0.070 inches (most preferably about 0.062 inches). As best seen in FIG. 4, cap 16, contains sealing surface 42 defined by tubular sidewall 44. Sidewall 44 also defines hollow bore 45 that is sized and shaped to be received over hub 20 so that sealing surface 42 contacts proximal face 28. Sealing surface 42 has a depth of between about 0.016 - 0.020 inches (most preferably about 0.018 inches). Cap 16 contains opening 49 opposite bore 45 that communicates with bore 45. Opening 49 is defined by proximal surface 17 that is roughly funnel shaped and sloped toward opening 49 and cavity 24.

Sealing disc 14 is roughly circular, contains cut 40, and is sized and shaped to fit within rabbet 32 of hub 20. Sealing disc 14 preferably has a thickness of between about 0.005-0.015 inches (most preferably about 0.010 inches). Sealing disc 14 may be made from any appropriate material, such as rubber or any suitable elastomer, but is most preferably made from a silicone rubber, such as Silastic^{®} silicone rubber sold by Dow Coming Corporation, Midland, Michigan. Cut 40 is located in the approximate center of sealing disc 14 entirely or partially across sealing disc 14 and extends entirely through the thickness of sealing disc 14. Cut 40 preferably is made at an angle of between about 40 - 50 degrees (most preferably 45 degrees) but any suitable angle may be used. Sealing disc 14 is seated within rabbet 32 of hub 20. Cavity 45 of cap 16 fits over hub 20 and slightly compresses sealing disc 14 such as between approximately 0.001-0.003 inches (most preferably about 0.002 inches). Cap 16 may be held in place by any appropriate mechanism, such as crimping or adhesive, but is most preferably held in place by interference or frictional fit between tubular sidewall 44 of cap 16 and hub 20.

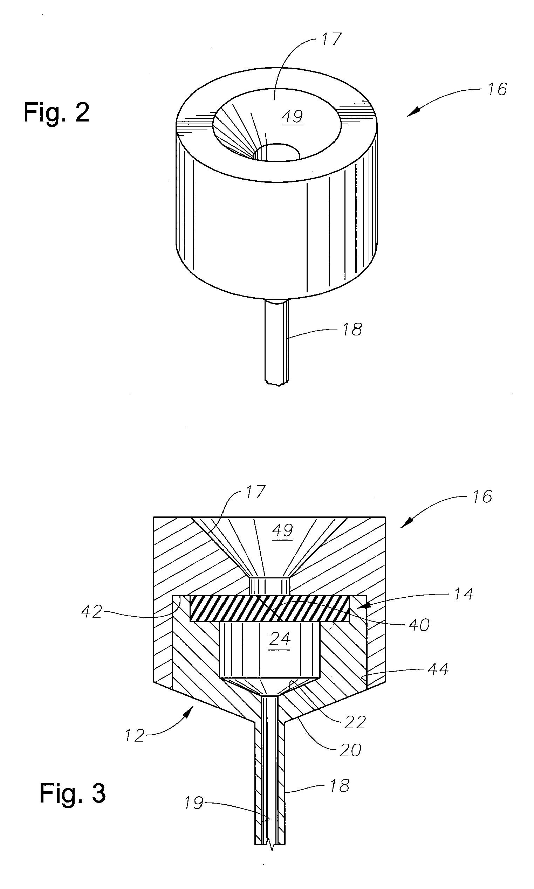

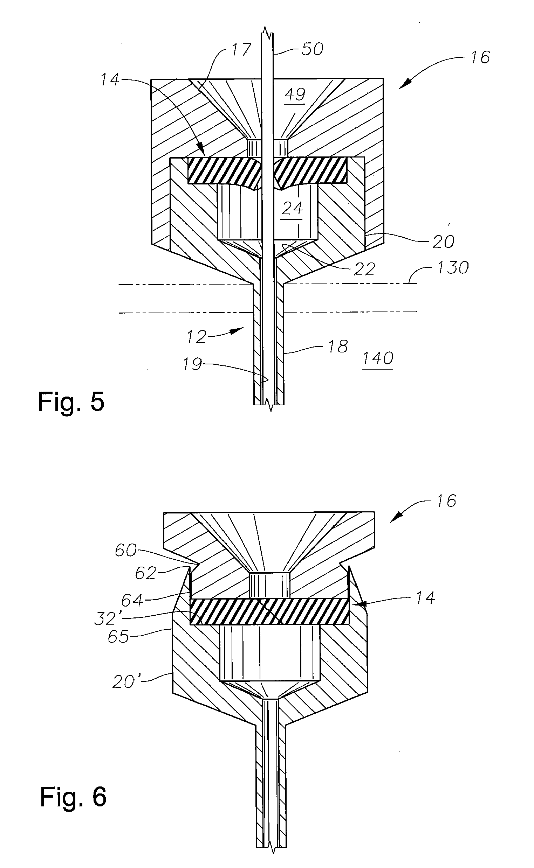

During operation, as best shown in FIG. 5, tube 18 is inserted through sclera 130. Microsurgical instrument 50 is inserted through opening 49, cut 40, cavity 24, tube 18, and into posterior chamber 140. The funnel shape of surface 17 of cap 16, and distal floor 22 of cavity 24, helps direct surgical instrument 50 into bore 19. Cavity 24 allows room for sealing disc 14 to deform inwardly without impeding the motion of, or increasing the friction on, surgical instrument 50. When the surgeon wishes to withdraw or exchange instruments, surgical instrument 50 is withdrawn from cannula 10. Cut 40 returns to its original closed position, thereby sealing tube 18, as seen in FIG. 3. The angle of cut 40 helps to seal sealing disc 14 and prevent loss of fluid and tissue.

In a second embodiment, shown in FIG. 6, hub 20' is of construction similar to hub 20 and is generally cylindrical and contains rabbet 32', which is deeper than rabbet 32 and of sufficient depth to receive both sealing disc 14 and cap 16'. Edge 62 extends proximally from hub 20' and may comprise a continuous flange around the circumference of hub 20', or may comprise a plurality of flanges disposed at regular or irregular intervals around the circumference of hub 20'. Edge 62 may be of any appropriate geometry, but is most preferably an angled or curved cut made in the proximal portion of sidewall 65 of hub 20'. Cap 16' is generally cylindrical, and has groove 60 in the circumference of outer wall 64. Cap 16' is received within rabbet 32' of hub 20', proximal sealing disc 14, thereby holding sealing disc 14 in place. Cap 16' slightly compresses sealing disc 14, and cap 16' is held in place by folding, crimping, or bending edge 62 into groove 60.

While certain embodiments of the present invention have been described above, these descriptions are given for purposes of illustration and explanation. Variations, changes, modifications and departures from the systems and methods disclosed above may be adopted without departure from the scope or spirit of the present invention.

## Claims

1. A cannula (10), comprising:
a) a body (12) comprising a tube (18) and a hub (20), the hub having a face (28) with a rabbet (32) opposite the tube, and an internal cavity (24), the cavity fluidly connected to the tube;
b) a sealing disc (14), disposed within the rabbet (32), the sealing disc having a cut (40); and
c) a cap (16) having an opening (49) and adapted to be received by the hub (20), the cap holding the sealing disc (14) within the rabbet, the opening providing access to the sealing disc.

2. The cannula of claim 1, wherein the cap (16) is received on the hub (20).

3. The cannula of claim 1, wherein the cap (16) is received over the hub (20).

4. The cannula of claim 1, wherein the cap (16') is received within the hub (20').

5. The cannula of any one of claims 1 to 4, claim 1 wherein the cut (40) is made at an angle.

6. The cannula of any one of claims 1 to 5, wherein the opening in the cap (16) communicates with the tube (18) through the sealing disc (14).

7. The cannula of any one of claims 1 to 6, wherein the cap (16) further comprises a funnel-shaped proximal surface (17).

8. The cannula of any one of claims 1 to 7, wherein the internal cavity (24) comprises a funnel-shaped distal face (22).

9. The cannula of any one of claims 1 to 8, wherein the body (12) and/or the cap (16) is/are made from surgical stainless steel, titanium, or thermoplastic.

10. The cannula of any one of claims 1 to 8, wherein the sealing disc (14) is made from silicone rubber, or an elastomer.
